# EUROPEAN PATENT APPLICATION

(11) **EP 1 729 134 A1**
(43) Date of publication of application: **06.12.2006**
(21) Application number: 06114359.0
(22) Date of filing: 23.05.2006
(51) Int. Cl.: G01N 33/68

(54) **In situ biomarker identification**

(30) Priority: 03.06.2005 EP 05104829; 21.03.2006 EP 06111461
(71) Applicant: F. HOFFMANN-LA ROCHE AG, 4070 Basel (CH)
(72) Inventor: Augustin, Angelique, 4125 Riehen (CH); Ducret, Axel, 4052 Basel (CH); Langen, Hanno, 79540 Loerrach (DE); Meistermann, Helene, 68100 Mulhouse (FR)

(57) **Abstract**

The invention relates to a method for identifying protein biomarkers directly in a specific region of interest of a tissue which comprises the identification of a mass of interest by analysis of MALDI imaging mass spectrometry results, followed by the identification of the protein represented by said mass of interest by direct elution from the specific region of interest of a tissue, fractionation and tandem mass spectrometry.

SEQUENCE LISTING

<110> F. Hoffmann-La Roche AG

<120> In situ biomarker identification

<130> case 23148

<160> 2

<170> PatentIn version 3.3

<210> 1

<211> 147

<212> PRT

<213> Rattus norvegicus

<220>

<221> transthyretin

<222> (1)..(147)

<223> swissprot: locus TTHY_RAT, accession P02767

<400> 1

Met Ala Ser Leu Arg Leu Phe Leu Leu Cys Leu Ala Gly Leu Ile Phe

1               5                   10                  15

Ala Ser Glu Ala Gly Pro Gly Gly Ala Gly Glu Ser Lys Cys Pro Leu

            20                  25                  30

Met Val Lys Val Leu Asp Ala Val Arg Gly Ser Pro Ala Val Asp Val
        35                  40                  45
Ala Val Lys Val Phe Lys Lys Thr Ala Asp Gly Ser Trp Glu Pro Phe
    50                  55                  60
Ala Ser Gly Lys Thr Ala Glu Ser Gly Glu Leu His Gly Leu Thr Thr
65                  70                  75                  80
Asp Glu Lys Phe Thr Glu Gly Val Tyr Arg Val Glu Leu Asp Thr Lys
                85                  90                  95
Ser Tyr Trp Lys Ala Leu Gly Ile Ser Pro Phe His Glu Tyr Ala Glu
            100                 105                 110
Val Val Phe Thr Ala Asn Asp Ser Gly His Arg His Tyr Thr Ile Ala
        115                 120                 125
Ala Leu Leu Ser Pro Tyr Ser Tyr Ser Thr Thr Ala Val Val Ser Asn
    130                 135                 140
Pro Gln Asn
145

<210> 2
<211> 120
<212> PRT
<213> Rattus norvegicus

<220>
<221> Ser 28 to Gln 146 processed form

<222>  (1)..(120)

<400>  2

Ser Lys Cys Pro Leu Met Val Lys Val Leu Asp Ala Val Arg Gly Ser
1               5                   10                  15

Pro Ala Val Asp Val Ala Val Lys Val Phe Lys Lys Thr Ala Asp Gly
            20                  25                  30

Ser Trp Glu Pro Phe Ala Ser Gly Lys Thr Ala Glu Ser Gly Glu Leu
        35                  40                  45

His Gly Leu Thr Thr Asp Glu Lys Phe Thr Glu Gly Val Tyr Arg Val
    50                  55                  60

Glu Leu Asp Thr Lys Ser Tyr Trp Lys Ala Leu Gly Ile Ser Pro Phe
65                  70                  75                  80

His Glu Tyr Ala Glu Val Val Phe Thr Ala Asn Asp Ser Gly His Arg
                85                  90                  95

His Tyr Thr Ile Ala Ala Leu Leu Ser Pro Tyr Ser Tyr Ser Thr Thr
            100                 105                 110

Ala Val Val Ser Asn Pro Gln Asn
        115                 120

## Description

The present invention relates to de novo in situ protein biomarker identification.

MALDI imaging mass spectrometry (IMS) represents a new technique for the direct peptide and protein analysis from a tissue thin section by conventional MALDI-TOF MS instruments (Chaurand. P, Schwartz, S.A, Billheimer, D., Xu, B.J., Crecelius, A Caprioli, R.M. (2004) Anal. Chem. 76, 1145-1155). The generated two-dimensional ion density map can then be used to derive the relative abundance and spatial distribution of proteins *in situ.* The usefulness of this technique has been demonstrated in the biomarker field in experimental models (Pierson, J., Norris, J. L., Aerni, H.-R., Svenningsson, P., Caprioli, R. M., and Andrén, P. E. (2004) Journal of Proteome Research 3, 289-295, Chaurand, P., DaGue, B. B., Pearsall, R. S., Threadgill, D. W., and Caprioli, R. M. (2001) Proteomics 1, 1320-1326) and in clinical settings, for instance for accurate and sensitive classification of non-small-cell lung cancer tumor biopsies (Yanagisawa, K., Shyr, Y., Xu, B. J., Massion, P. P., Larsen, P. H., White, B. C., Roberts, J. R., Edgerton, M., Gonzales, A., Nadaf, S., Moore, J. H., Caprioli, R. M., and Carbone, D. P. (2003) The Lancet 362, 433-439).

Currently known methods for identification of de novo protein biomarkers in diseased tissues by MALDI MS involves tissue homogenization followed by one- or two dimensional electrophoresis and identification of modulated protein levels by MALDI MS.

The present invention provides a novel method for de novo in situ protein biomarker identification wherein proteins are directly eluted from the tissue region of interest without any need for tissue homogenization.
Proteomics techniques have the potential to correlate differential protein expression with environmental disease or toxicant exposure. High resolution two-dimensional gel electrophoresis technology in combination with mass spectrometry have demonstrated the usefulness of proteomics approaches especially in the toxicology area (Bandara, L. R., and Kennedy, S. (2002) Drug Discovery Today 7, 411-418). Of particular interest is the early detection of disease but also the rapid screening against experimental compounds either for toxicity or efficacy (Petricoin, E. F., Rajapaske, V., Herman, E., Arekani, A M., Ross, S., Johann, D., Knapton, A., Zhang, J., Hitt, B. A., Conrads, T. P., Veenstra, T. D., Liotta, L. A., and Sistare, F. D. (2004) Toxicologic Pathology 32, 122-130). The feasibility of toxicoproteomics studies has been demonstrated both for the identification of predictive markers and for the elucidation of toxicant mode (Witzmann, F. A., and Li, J. (2004) Proteomics in Nephrology 141, 104-123; Bandara, L. R., Kelly, M. D., Lock, E. A., and Kennedy, S. (2003) Toxicological Sciences 73, 195-206).

MALDI imaging mass spectrometry (IMS) represents a new technique for the direct peptide and protein analysis from a tissue thin section by conventional MALDI-TOF MS instruments (Chaurand. P, Schwartz, S.A, Billheimer, D., Xu, B.J., Crecelius, A. Caprioli, R.M. (2004) Anal. Chem. 76, 1145-1155). The generated two-dimensional ion density map can then be used to derive the relative abundance and spatial distribution of proteins *in situ.* The usefulness of this technique has been demonstrated in the biomarker field in experimental models (Pierson, J., Norris, J. L., Aerni, H.-R., Svenningsson, P., Caprioli, R. M., and Andrén, P. E. (2004) Journal of Proteome Research 3, 289-295, Chaurand, P., DaGue, B. B., Pearsall, R. S., Threadgill, D. W., and Caprioli, R. M. (2001) Proteomics 1, 1320-1326) and in clinical settings, for instance for accurate and sensitive classification of non-small-cell lung cancer tumor biopsies (Yanagisawa, K, Shyr, Y., Xu, B. J., Massion, P. P., Larsen, P. H., White, B. C., Roberts, J. R., Edgerton, M., Gonzales, A., Nadaf, S., Moore, J. H., Caprioli, R. M., and Carbone, D. P. (2003) The Lancet 362, 433-439).

According to the present invention a method is provided for de novo in situ identification of protein biomarkers by the novel combination of MALDI IMS with associated techniques.

In a first step of the present invention, a tissue thin section from a specific tissue of interest is provided. Preferably, said tissue thin section is 5 to 15 µm thick. The tissue section can be further processed, e.g. by fixation with an alcohol, acetonitrile etc. Preferably, the fixative is ethanol. Then, a MALDI matrix is deposited on said tissue section.

Said tissue section can originate from any tissue of interest of any multicellular organism. For example, such tissue can originate from a human subject, any non-human multicellular organism, including non-human mammal, e.g. mouse, rat, rabbit, swine, non-human primate, but also from other organisms such as e.g. insects, nematodes or plants.

Suitable MALDI matrices are well known in the art (see e.g. Trauger et al., Spectroscopy 2002, 16(1), 15-28). Some MALDI matrices are more suitable for peptide analysis (e.g. alpha cyano cinnamic acid), but they are not optimal for protein applications. Preferably, said MALDI matrix is a matrix suitable for protein applications, such as sinnapinic acid as a non limiting example.

In a further step, MALDI MS spectra are acquired by exposing the tissue section to a laser beam. Laser beams suitable to generate MALDI MS spectra are well known in the art. As a non limiting example, a standard 337-nm N₂ laser operating at 50 Hz can be used. Preferably, said laser has a laser spot size of 10 to 100, more preferably of 40 to 60, most preferably of 50 µm.

The MALDI MS spectra generated above were then analyzed.

Tools for analysis of MALDI MS spectra are well known to the skilled person and can be obtained commercially. One such tool is e.g. the ProTS-Data software from Efeckta Technologies Inc. Peaks of interest are then selected. Such selection may, as a non-limiting example, be based on a ranking of spectral features according to the extent of observed differences, e.g. using the Weighted Flexible Compound Covariate Method (WFCCM) (Shyr, Y., and KyungMann, K (2003) A Practical Approach to Microarray Data Analysis, ed D. Berrar*).*

In a further step of the method of the present invention, proteins are eluted from a tissue sample corresponding to the tissue thin section hereinbefore described. Said tissue sample may be a large piece of tissue or a microdissected tissue. Preferably, said tissue sample is a tissue thin section corresponding to the tissue thin section used for MALDI MS analysis hereinbefore described. Said tissue thin section can also be the same tissue thin section as the one used for MALDI MS in the previous steps. The elution of proteins is achieved by protein micro-extraction, which can be achieved, as a non-limiting example, by directly pipetting up and down a small volume of extraction solvent on the region of interest of the tissue section. This process can be repeated several times and samples be pooled to obtain enough material for subsequent protein identification.

In a next step, the eluates are fractionated. Fractionation can be performed using HPLC.

The fraction containing the mass (peak) of interest is identified by MALDI TOF MS.

The protein contained in said fraction is then identified by performing tandem mass spectrometry and analyzing the resulting spectrum. As a non-limiting example, the protein can be identified with the Mascot MS/MS ion search program (MatrixScience) using the SwissProt database.

The accumulation of the protein transthyretin identified in the kidney cortex in nephrotoxicity according to the example provided below is a processed protein which accumulates in a tissue where it is not intrinsically expressed, therefore, it would not be possible to identify said biomarker by methods based on DNA expression.

### Short description of Figures:

Figure 1 Matrix spotting strategy on kidney thin section
Figure 2 Cluster analysis of the 7 most discriminating features in the cortex of control versus gentamicin-treated rat kidney
Figure 3 Average signal of m/z 12959 in the cortex of control and gentamicin-treated rat kidney
Fig 4 Identification strategy
Fig 5 Tandem mass spectrometric analysis of m/z 12959 A) Full mass spectrum. B) Deconvulated mass spectrum. C) Annotated tandem mass spectrum
Fig 6 Database search result
Fig 7 A) Western Blot analysis of the cytosolic fractions from kidney cortex and from HPLC fraction D10. B) MALDI MS analysis of rat kidney cortex and of HPLC fraction D10G/C: HPLC fraction D10 gentamicin/control; G/C: kidney cytosolic fraction gentamicin/control

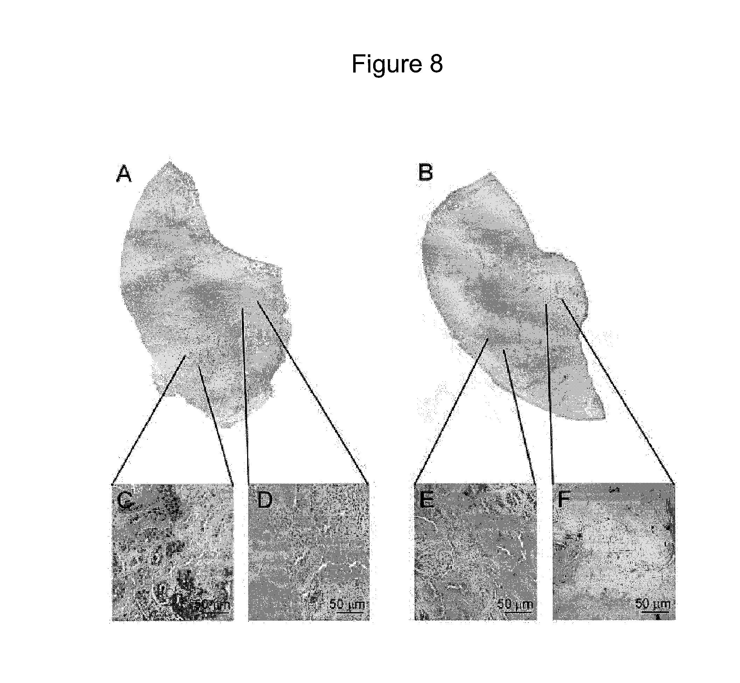

Fig 8: Immunohistochemical examination of kidney from control and gentamicin-treated rat. Scanned digitized images of whole kidney sections from a treated (A) and a control (B) rat show high levels of transthyretin immunoreactivity in the treated kidney cortex. High magnification photomicrographs from the cortex (C, E) vs. the medulla (D, F) reveal specific localization of TTR immunoreactivity in the cortex tubules of the treated kidney. Transthyretin immunoreactivity is visualized by the grey precipitate of diaminobenzidine-Ni counterstained with cresyl violet.

### Examples

### Example 1: Model system for nephrotoxicity

### Example 1a): Animal Treatment.

Permission for animal studies was obtained from the local regulatory agencies, and all study protocols were in compliance with animal welfare guidelines. Male HanBrl:
Wistar rats of approximately 12 weeks of age (300g ± 20%) were obtained from BRL, Füllinsdorf, Switzerland. The animals were housed individually in Macrolone cages with wood shavings as bedding at 20°C and 50% relative humidity in a 12-hr light/dark rhythm with free access to water and Kliba 3433 rodent pellets (Provimi Kliba AG, Kaiseraugst, Switzerland).

Animals were treated for 7 consecutive days with 100 mg/kg/day Gentamicin (dissolved in saline) or vehicle by subcutaneous (sc) injections and sacrificed 24 hours after the last application by CO₂ inhalation. Immediately preceding sacrifice, terminal blood samples for clinical chemistry investigations were collected from the retroorbital sinus under isofluoran anesthesia.

### Example 1 b): Histology.

Representative kidney samples were fixed in 10% neutral buffered formalin. All samples were processed using routine procedures and embedded in Paraplast. Tissue sections approximately 2-3 microns were cut and stained with hematoxylin-eosin (HE) or periodic acid-Schiff (PAS).

### Example 2: MALDI-IMS of rat kidney cortex treated with gentamicin

The differential protein expression was studied within the areas of the kidney (Cortex, Medulla, Papilla) to investigate whether the histopathologically-observed toxicity of gentamicin could be correlated by IMS. Several protein peaks in the cortex area were significantly modulated upon gentamicin administration.

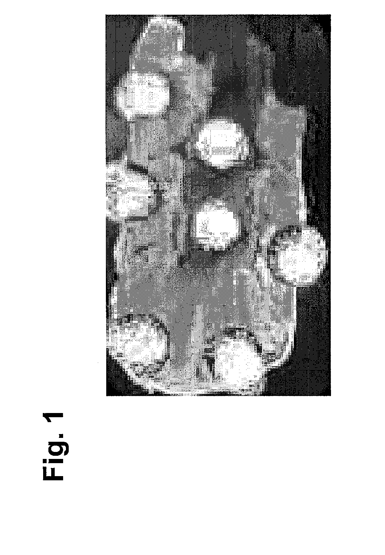

Marked degeneration/regeneration of proximal tubules (grade 4) was clearly observed in rats treated with 100 mg/kg/day gentamicin for 7 days. This finding was correlated to the analysis of rat kidney thin sections by MALDI IMS. 9 control and 9 gentamicin-treated rat kidneys thin sections (3 animals, 3 sections from each animal; the thin section was spotted with matrix as shown in Fig. 1) were subjected to mass spectrometric analysis as described below.

### Example 2 a): Sample Preparation for MALDI IMS.

Kidneys were dissected from rats, snap frozen on dry ice and stored at -80°C prior to further processing. 12 µm sections were obtained on a cryostat (LEICACM 3000, Leica Microsystems) at -18°C and deposited on ITO-coated conductive glass slides (Indium Tin Oxide 50 x 75 x 0.9 mm, Delta Technologies). Sample preparation were performed according to published procedure (Schwartz, S. A., Reyzer, M. L., and Caprioli, R. M. (2003) Journal of Mass Spectrometry 38, 699-708). Tissue sections were then fixed by immersion in ethanol bathes and let to dry 30 minutes under vacuum. During all the process the sections were stored as much as possible under vacuum. Deposition of MALDI matrix was done manually with a pipette. 200 nL of freshly prepared sinapinic acid solution (3,5-dimethoxy-4-hydroxycinnaminic acid, Fluka, 20 mg/ml in water/acetonitrile 1:1, 0.1% trifluoroacetic acid) was deposited onto the tissue. After crystallization, an additional 200 nL was layered onto the spots and the matrix was let to dry at room temperature. The sample was then kept under vacuum if not analyzed immediately by mass spectrometry.

Example 2 b): Mass Spectrometry.

MALDI MS spectra were acquired using an UltraFlex MALDI ToF/ToF instrument (Bruker Daltonics) with a standard 337-nm N₂ laser operating at 50Hz and a laser spot size of 50 µm. The instrument was operated in positive linear mode (2kDa-30kDa) at constant laser power. A total of 8 times 100 laser shots were averaged for each manually deposited matrix droplets. Spectra were externally calibrated using a protein calibration standard solution (Insulin, Ubiquitin I, Cytochrom C, Myoglobin; Bruker Daltonics). Internal calibration was performed post-acquisition using redundant known peaks from the spectra (Hemoglobin α-chain, Thymosin β-4, Cytochrome c oxidase polypeptide VIIa L, Ubiquitin, Cytochrome c oxidase polypeptide VIb).

### Example 2 c): Data Analysis.

The spectra were processed using a combination of tools available commercially and developed in-house at Vanderbilt University. Baseline subtraction, normalization, peak detection, and spectral alignment were performed using the ProTS-Data software (Efeckta Technologies, Inc.) All spectra were baseline corrected to remove contributions of chemical noise to the signal. This step was performed by locally estimating the baseline and subtracting the spectrum from the raw data. For the present data set the software was set to consider a window width 10 times that of the peak width to account for the differences in resolution for different m/z regions. The baseline corrected spectra were normalized in intensity by scaling the spectra to a common total ion current. Peak detection was performed using ProTS-Data and a list of 13 common peaks was selected to be internal alignment points using a quadratic calibration function. The criterion used to select common peaks were that the peak must occur in greater than 80 % of the spectra to be aligned, must not have interfering or overlapping peaks, and the peaks must have a standard deviation of the observed centroid value less that 7 mass units. To avoid error caused by extrapolation during the alignment step, alignment peaks were chosen across the entire mass range of interest ranging from 2500 to 15000 mass units. The peak lists containing the centroid value and normalized intensity are exported to individual files.

The peak lists were binned according to their m/z value using an in-house program developed at Vanderbilt University. The exported MALDI-TOF MS peaks were aligned across samples by use of a genetic algorithm parallel search strategy (Yanagisawa, K., Shyr, Y., Xu, B. J., Massion, P. P., Larsen, P. H., White, B. C., Roberts, J. R., Edgerton, M., Gonzales, A., Nadaf, S., Moore, J. H., Caprioli, R. M., and Carbone, D. P. (2003) The Lancet 362, 433-439). Briefly, peaks were binned together such that the number of peaks in a bin from different samples is maximized while the number of peaks in a bin from the same sample is minimized. A series of mass windows or peak bins were generated that separated similar peaks across multiple spectra. The spectral features were ranked according to the extent of the observed difference in order to determine relevant biomarkers for gentamicin induced kidney toxicity using the Weighted Flexible Compound Covariate Method (WFCCM). This strategy, described by Shyr *et al*.(Shyr, Y., and KyungMann, K (2003) A Practical Approach to Microarray Data Analysis, ed D. Berrar), uses a combination of 6 different statistical tests to compute a ranking based on the difference observed between treated and control.

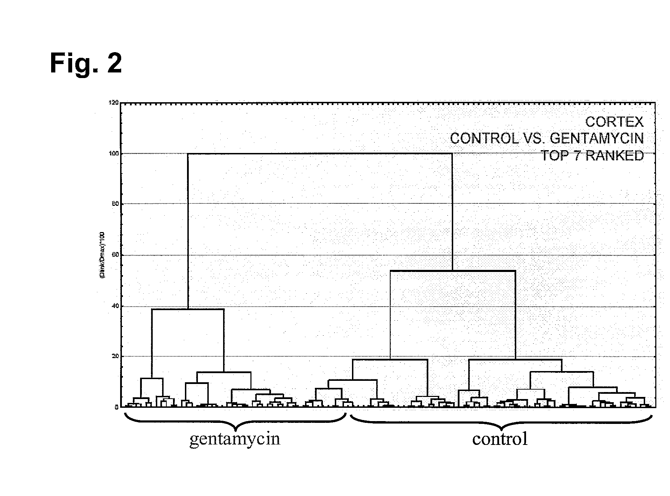

A simple data cluster analysis was successful in differentiating the control from the treated conditions. As expected, most of the peaks discriminating the control from the treated were found in the cortex. Table 1 shows the proteins that were statistically identified as being up or down regulated in the control versus the treated samples in the cortex. Fig 2 shows the cluster that can be obtained using the 7 top ranked peaks based on the WMA, the maximum average S/N and the p-value.

**Table 1: Cortex profiling: Biomarkers ranking for gentamicin-induced kidney toxicity**

| | Weighted Means Average | p-value | Average S/N (gent) | Average S/N (control) | Maximum Average S/N |
|---|---|---|---|---|---|
| 12959.23 | 1.5 | 2.E-14 | 5 | 0 | 5 |
| 6498.996 | -1.1 | 2.E-11 | 0 | 2 | 2 |
| 4497.792 | -0.9 | 9.E-11 | 0 | 5 | 5 |
| 6478.957 | 0.9 | 5.E-07 | 2 | 0 | 2 |
| 7083.364 | 0.8 | 2.E-10 | 6 | 1 | 6 |
| 4107.083 | 0.8 | 9.E-07 | 5 | 0 | 5 |
| 5222.249 | -0.8 | 1.E-07 | 0 | 2 | 2 |
| 5096.886 | -0.7 | 2.E-11 | 5 | 11 | 11 |
| 4010.498 | -0.7 | 8.E-11 | 1 | 3 | 3 |
| 4141.907 | -0.7 | 2.E-10 | 1 | 3 | 3 |
| 5355.689 | -0.7 | 2.E-10 | 1 | 3 | 3 |
| 13004.71 | -0.7 | 6.E-09 | 0 | 3 | 3 |
| 17176.83 | -0.7 | 7.E-09 | 0 | 2 | 2 |
| 17100.68 | -0.7 | 1.E-06 | 0 | 2 | 2 |
| 6938.997 | 0.7 | 4.E-05 | 1 | 0 | 1 |
| 6912.956 | -0.6 | 2.E-09 | 6 | 10 | 10 |
| 3343.714 | -0.6 | 2.E-07 | 2 | 5 | 5 |
| 5301.652 | -0.6 | 4.E-07 | 0 | 3 | 3 |
| 4181.504 | -0.6 | 9.E-08 | 0 | 2 | 2 |
| 6080.035 | 0.6 | 6.E-04 | 1 | 0 | 1 |

Ranking by:
1) WMA
2) Maximum average S/N
3) p-value

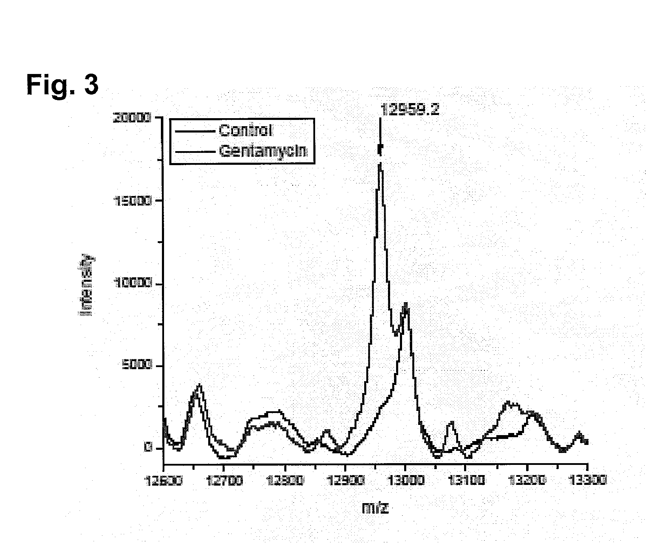

In this dataset, a peak at mass m/z 12 959 was ranked in 1^{st} position in the cortex with an average signal-over-noise of 5 in gentamicin-treated rat kidney and completely absent in the controls (Fig. 3). The doubly-charged species at m/z 6480 showed a similar behavior.

### Example 3: Microelution

A liquid micro-extraction was performed in the cortex area.

Protein micro-extraction was performed by directly pipetting up and down for a few seconds 1 µl of solvent (water/acetonitrile 1:1, 0.1% trifluoroacetic acid) on the cortex area of the kidney. This process was repeated several times on several samples to obtain a pooled sample volume of approximately 20 µl. The sample was then dried in a speed vac and resolubilized in water/acetonitrile 95:5, 0.1% trifluoroacetic acid.

The resulting protein mixture was fractionated by reverse-phase liquid chromatography.

Protein fractionation was performed using a standard Agilent 1100 series HPLC (Agilent Technologies) equipped with a 1 mm i.d. polymeric column (219TP5110, Vydac) operated at 50 µl/min. Solvent A was 0.1% trifluoroacetic acid in water and solvent B was 0.1% trifluoroacetic acid in acetonitrile. Proteins were eluted using the following program: the gradient was started after 5 minutes with 5%B up to 35% in 7 minutes, then to 60%B in 34 minutes, and then increased to 90%B in 0.5 minute and hold for 10 minutes. Fractions were collected every 30 sec by time immediately after the beginning of the run up to 48 minutes directly in a 96 well microtiterplate (Greiner bio-one). If necessary, a sample was fractionated over several HPLC runs and corresponding fractions were then combined for analysis.

The obtained LC fractions were surveyed by MALDI-TOF MS by spotting 1 µl of the fraction with 1 µl of 1 g/L sinapinic acid solution in water/acetonitrile 90:10, 0.1%TFA on a 384 MALDI Anchor target (Bruker Daltonics).

The presence of the peaks of interest was assessed by comparing the IMS-generated spectra with the LC-fractionated spectra.

### Example 3 a): Protein Identification.

The fraction containing the protein species of interest was then further analyzed by nano-ESI mass spectrometry and the proteins of interest were identified by tandem mass spectrometry.

For protein identification, the HPLC fraction containing the mass of interest was dried in a speed vac and resolubilized in water/acetonitrile 1:1 containing 1% formic acid for identification using an Applied Biosystems Q-Star Pulsar operated under standard operating conditions in nano-electrospray mode. One of the multiply charged species of the mass of interest was selected for tandem mass spectrometric analysis, its tandem mass spectrum was recorded and manually interpreted. Protein identification was performed with the Mascot MS/MS ion search program (Matrix Science, http://www.matrixscience.com) using the SwissProt database (release 46.6) and a tolerance of 1.0 Da for fragment and precursor masses.

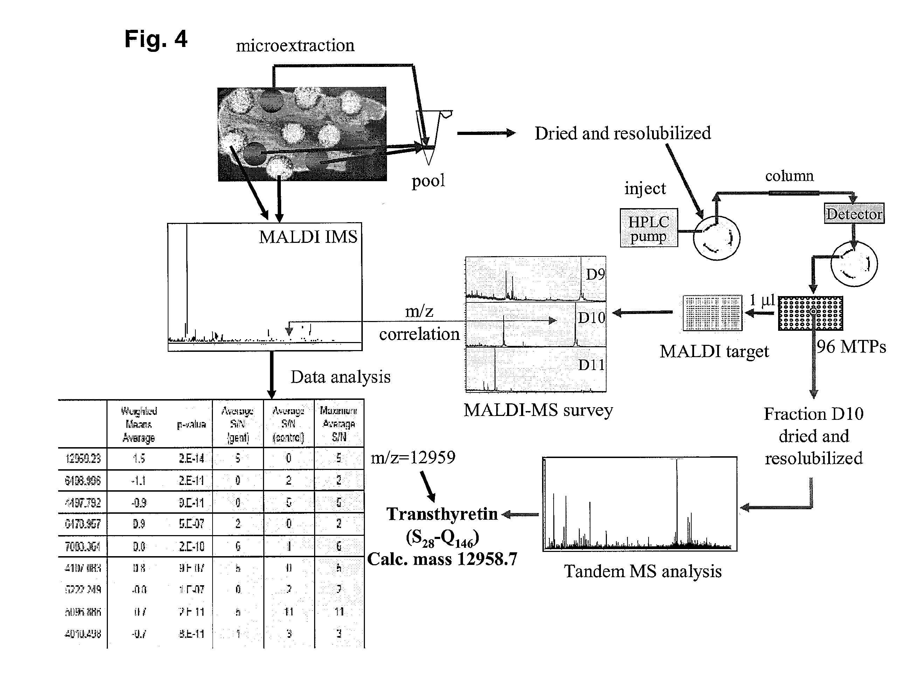

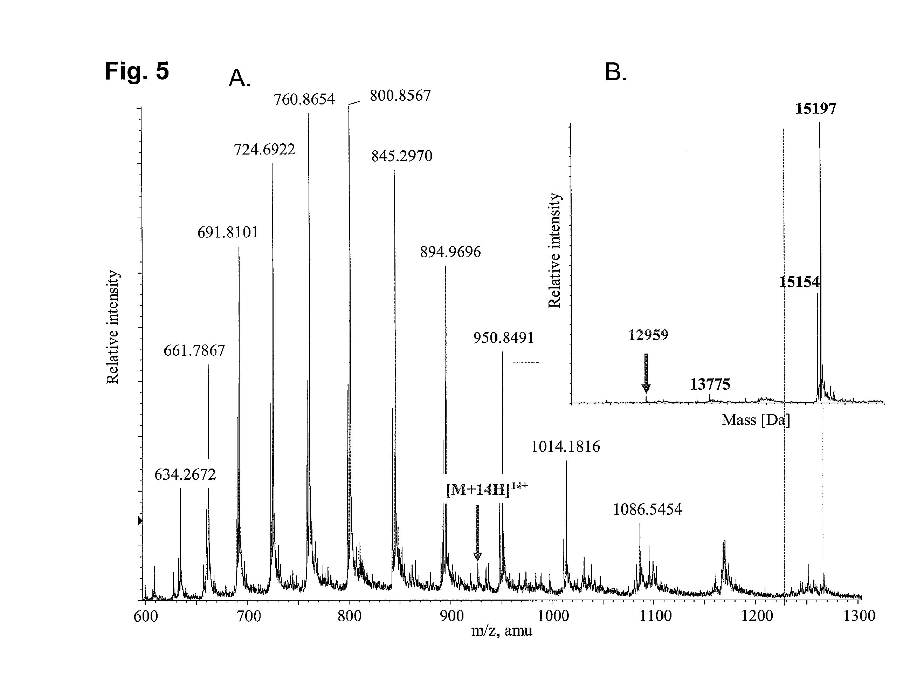

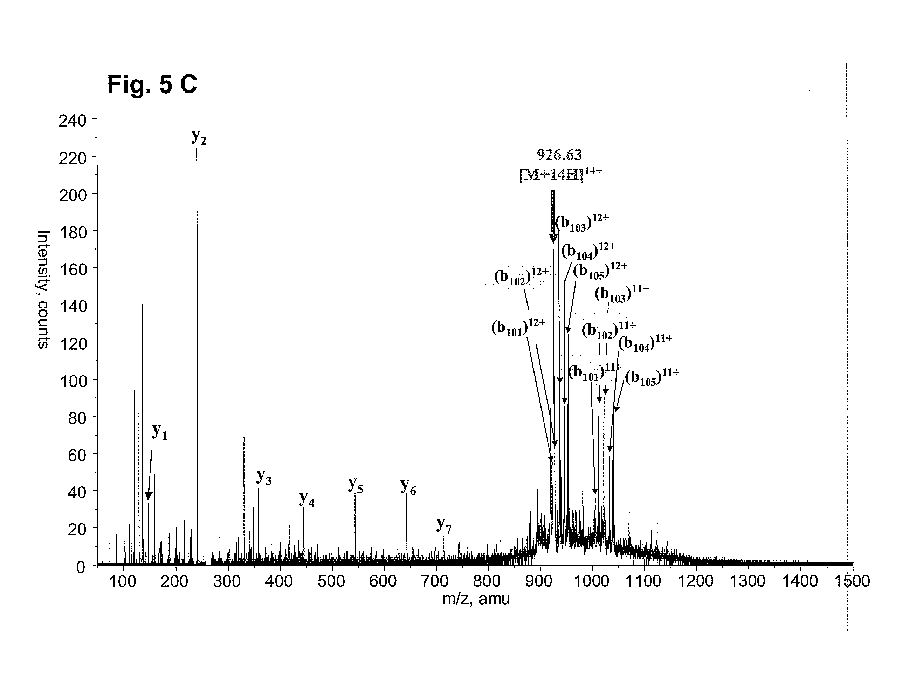

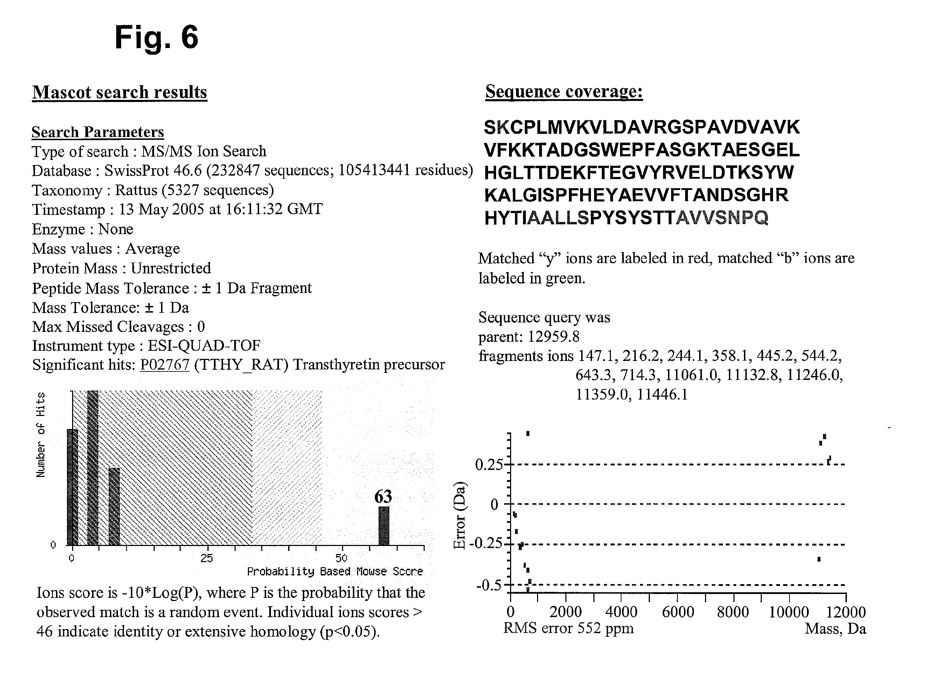

The strategy that was followed to identify this peak is illustrated in Fig. 4. Proteins were micro-extracted from the cortex area and were fractionated by reverse phase chromatography. A protein of m/z 12959 was found in the fraction D10 and could be successfully aligned with the species detected in the spectrum recorded directly from the tissue sample. The fraction was dried and reconstituted in 50% acetonitrile, 1% formic acid for tandem mass spectrometric analysis (Fig. 5). The generated mass spectrum could successfully be matched to transthyretin (sw:TTHY_RAT) from Ser28 to Gln146 (Fig. 6).

### Example 4: SDS-Polyacrylamide Gel Electrophoresis and Western Blot analysis of Tissue Extract and HPLC fraction.

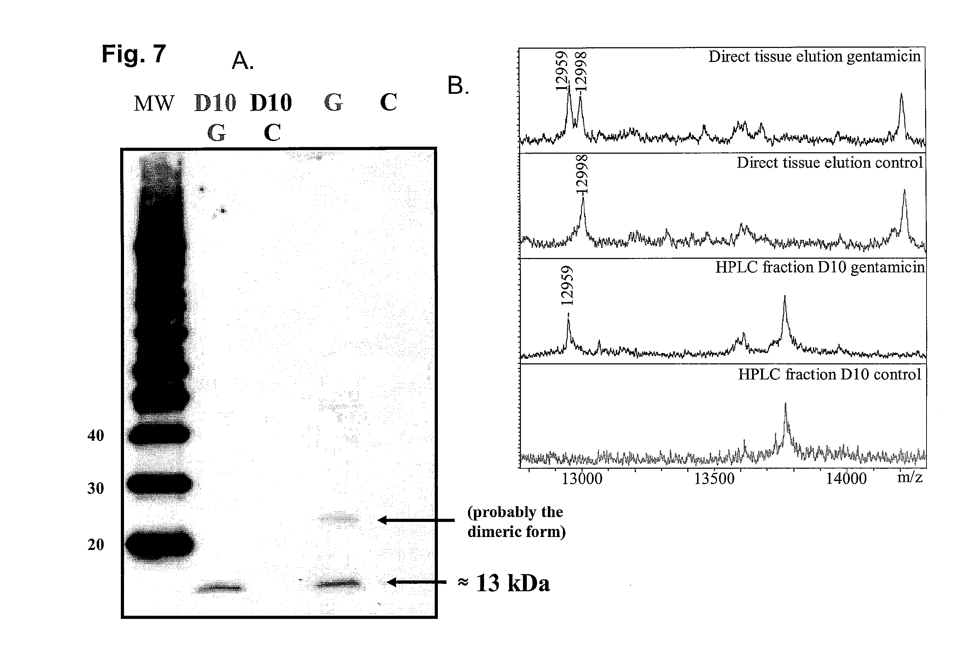

One of the biomarkers modulated upon gentamicin administration was identified as transthyretin (prealbumin), a 13 kDa blood transporter protein. This finding was confirmed by Western blot using an antibody highly specific for this protein showing that this protein was significantly more abundant in kidneys from gentamicin-treated rats than in control rats (Fig. 7).

Tissue extracts from control and treated rat kidneys were obtained by homogenization of a piece of cortex in a 10 mM Tris-HCl buffer pH 7.6 containing 250 mM sucrose and protease inhibitors (Roche Complete). The tissue extract was subjected to three successive centrifugation steps at 4 °C (10 min at 680g; 10 min at 10 000g; 1 h at 100 000g) whereas the pellets were discarded and the supernatant of the last centrifugation step was kept as the kidney cytosolic fraction.

1 µl of the kidney cytosolic fraction and of the relevant HPLC fraction were subjected to electrophoresis using a 4-20% Tris-Glycine SDS-PAGE gel (Invitrogen) and the proteins were subsequently transferred to a PVDF membrane. Blots were blocked in 5% milk in PBS containing 0.1% Tween 20 for 1 h and incubated overnight at room temperature with an anti-sheep prealbumin antibody (Abcam) in PBS containing 5% milk and 0.1% Tween 20. After washing with PBS containing 0.1% Tween 20, blots were incubated for 1 h with horseradish peroxidase conjugated with an anti-sheep IgG antibody (Silenus Laboratories). Antibody binding was detected using ECL western blotting reagents (Amersham).

Similarly, the m/y species 12959, which was identified by tandem mass spectrometry as transthyretin S₂₈-Q₁₄₆, was only present in the mass spectrum of the gentamicin-treated sample *in situ* and in the HPLC fraction D10.

## Claims

1. A method to identify a polypeptide which is accumulated or depleted in situ in a specific tissue, comprising the steps of
a) Providing a tissue thin section;
b) Depositing a MALDI matrix on a tissue thin section;
c) Acquiring MALDI MS spectra by exposing the tissue thin section of step a) to a laser beam;
d) Analysing the peaks in said spectra;
e) Eluting proteins from a tissue sample corresponding to the tissue thin section of steps a) to c);
f) Fractionating the eluted polypeptides;
g) Identifying a fraction comprising a peak of interest according to step c) by MALDI MS;
h) Identifying the polypeptide corresponding to said peak by Tandem MS analysis.

2. The method of claim 1, wherein said tissue thin section is fixed.

3. The method of claims 1 or 2, wherein the tissue sample is a tissue thin section.

4. The method of any one of claims 1 to 3, wherein in step a) the MALDI matrix is deposited on a specific area of interest of the tissue section, and in step d) proteins are eluted from said specific area of interest of the tissue.

5. The method of any one of claims 1 to 4 wherein said eluting in step d) is performed by directly pipetting up and down a small volume of extraction solvent on the region of interest.

6. The method of any one of claims 1 to 5, wherein said specific tissue originates from a human subject.

7. The method of any one of claims 1 to 6, wherein said specific tissue originates from a non-human multicellular organism.

8. The method of any one of claims 1 to 7, wherein the laser beam has a laser spot size of 10 to 100 µm.
